# FASCICULE DE BREVET EUROPEEN

(11) **EP 1 147 090 B1**
(45) Date de publication et mention de la délivrance du brevet: **23.11.2005**
(21) Numéro de dépôt: 00900582.8
(22) Date de dépôt: 14.01.2000
(51) Int. Cl.: C07D 231/38, C07D 403/12, A61K 7/13

(54) **BASES D'OXYDATION CATIONIQUES, LEUR UTILISATION POUR LA TEINTURE D'OXYDATION DES FIBRES KERATINIQUES, COMPOSITIONS TINCTORIALES ET PROCEDES DE TEINTURE**
KATIONISCHE OXYDATIONSBASEN, IHRE VERWENDUNG ZUR OXIDATIVEN FÄRBUNG VON KERATINFASERN, FÄRBEZUBEREITUNGEN UND FÄRBEVERFAHREN
NOVEL CATIONIC OXIDATION BASES, THEIR USE FOR DYEING KERATIN FIBRES, DYEING COMPOSITIONS AND DYEING METHODS

(30) Priorité: 19.01.1999 FR 9900505
(43) Date de publication de la demande: 24.10.2001
(73) Titulaire: L'OREAL, 75008 Paris (FR)
(72) Inventeur: TERRANOVA, Eric, F-92270 Bois Colombes (FR); LAGRANGE, Alain, F-77700 Coupvray (FR)
(74) Mandataire: Fevrier, Murielle Françoise E.
(86) Numéro de dépôt international: PCT/FR2000/000074
(87) Numéro de publication internationale: WO 2000/043367

(56) Documents cités:
- EP-A- 0 740 931
- WO-A-97/42173

## Description

L'invention a pour objet de nouveaux dérivés pyrazoliques comportant au moins un groupement cationique Z, Z étant choisi parmi des chaînes aliphatiques quaternisées, des chaînes aliphatiques comportant au moins un cycle saturé quaternisé, et des chaînes aliphatiques comportant au moins un cycle insaturé quatemisé, leur utilisation à titre de base d'oxydation pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Or, la demanderesse vient maintenant de découvrir, de façon totalement inattendue et surprenante, que de nouveaux dérivés pyrazoliques de formule (I) ci-après définis comportant au moins un groupement cationique Z, Z étant choisi parmi des chaînes aliphatiques quatemisées, des chaînes aliphatiques comportant au moins un cycle saturé quatemisé, et des chaînes aliphatiques comportant au moins un cycle insaturé quaternisé, non seulement conviennent pour une utilisation comme base d'oxydation, mais en outre qu'ils permettent d'obtenir des compositions tinctoriales conduisant à des colorations puissantes. dans une large palette de couleurs, et présentant d'excellentes propriétés de résistances aux différents traitements que peuvent subir les fibres kératiniques.

Ces découvertes sont à la base de la présente invention.

L'invention a donc pour premier objet de nouveaux composés de formule (I) suivante, leurs sels d'addition avec un acide ou avec une base, et leurs possibles formes tautomères : 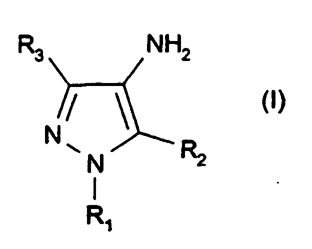 dans laquelle :
- R₁ représenté un atome d'hydrogène ; un groupement Z tel que défini ci-après ; un radical alkyle en C₁-C₆ ; un radical trifluoroalkyle en C₁-C₆ ; un radical monohydroxyalkyle en C₁-C₆ ; un radical polyhydroxyalkyle en C₂-C₆ ; un radical arylalkyle en C₁-C₆ dans lequel le radical aryle est un radical phényle ou un hétérocycle aromatique à cinq ou six chaînons tel que par exemple un cycle pyridyle, un cycle imidazolyle, un cycle furyle ou un cycle oxazolyle ; un radical alcoxy(C₁-C₆)alkyle en C₁-C₆ ; un radical alkyl(C₁-C₆)thio alkyle en C₁-C₆; un radical aminoalkyl(C₁-C₆)carbonylalkyle(C₁-C₆); un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical aminosulfonylalkyle(C₁-C₆) ; un radical N-alkyl(C₁-C₆) aminosulfonylalkyle(C₁-C₆) ; un radical N,N-dialkyl(C₁-C₆) aminosulfonylalkyle(C₁-C₆) ;
- R₂ et R₃, identiques ou différents représentent un groupement -NHR₄ ; un radical hydroxyle ; un atome d'halogène ; un radical nitro, un radical cyano ; un radical carboxy ; un radical carboxy alkyle en C₁-C₆ ; un radical carbamyle ; un radical N-alkyl(C₁-C₆)carbamyle ; un radical N,N-dialkyl(C₁-C₆)carbamyle; un radical alkoxy en C₁-C₆ ; un radical thioalkyle en C₁-C₆ ; ou l'une des significations données ci-dessus pour R₁ ; étant entendu qu'au moins un des radicaux R₂ et R₃ représente un groupement -NHR₄ ou un radical hydroxyle ;
- R₄ représente un atome d'hydrogène ; un groupement Z tel que défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C,-C₆)carbonyle, alkyl(C₁-C₆)sulfonyle, formyle, trifluoroalkyl(C₁-C₆)carbonyle, l'amine du radical aminoalkyle en C₁-C₆ peut également être substituée par deux radicaux formant, conjointement avec l'atome d'azote de ladite amine, un cycle saturé ou insaturé à cinq ou six chaînons et pouvant contenir un ou plusieurs hétéroatomes choisis parmi l'azote, l'oxygène et le soufre, tel que par exemple un cycle pipéridine, morpholine, imidazole ou oxazole ;
- Z est choisi parmi les groupements cationiques insaturés de formules (II) et (III) suivantes, et les groupements cationiques saturés de formule (IV) suivante : 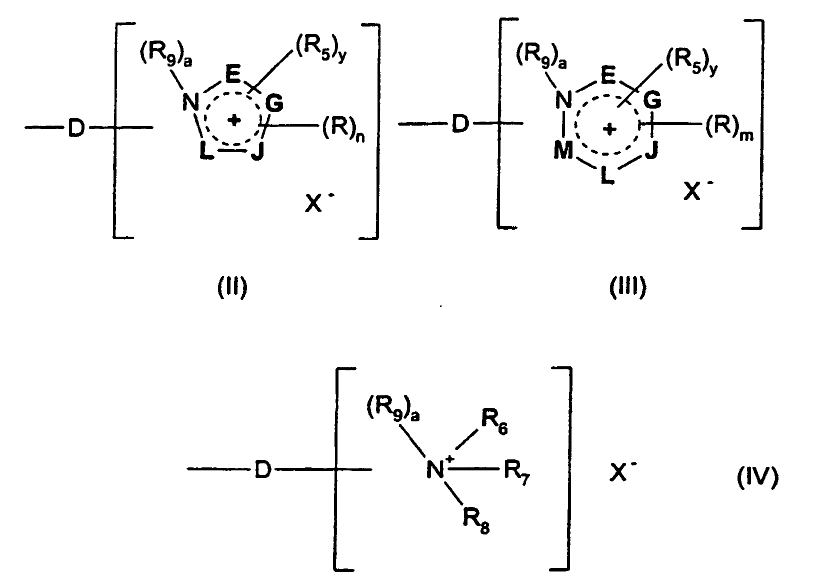 dans lesquelles :
   - D est un bras de liaison qui représente une chaîne alkyle comportant de préférence de 1 à 14 atomes de carbone, linéaire ou ramifiée, et pouvant être substituée par un ou plusieurs radicaux hydroxyle ou alcoxy en C₁-C₆, et pouvant porter une ou plusieurs fonctions cétone ;
   - les sommets E, G, J, L et M, identiques ou différents, représentent un atome de carbone ou d'azote ;
   - n est un nombre entier compris entre 0 et 4 inclusivement ;
   - m est un nombre entier compris entre 0 et 5 inclusivement ;
   - les radicaux R, identiques ou différents, représentent un atome d'halogène ; un radical hydroxyle ; un radical alkyle en C₁-C₆ ; un radical monohydroxyalkyle en C₁-C₆ ; un radical polyhydroxyalkyle en C₂-C₆ ; un radical nitro ; un radical cyano ; un radical cyanoalkyle en C₁-C₆ ; un radical alcoxy en C₁-C₆ ; un radical trialkyl(C₁-C₆)silanealkyle en C₁-C₆ ; un radical amido ; un radical aldéhydo ; un radical carboxyle ; un radical alkylcarbonyle en C₁-C₆ ; un radical thio ; un radical thioalkyle en C₁-C₆ ; un radical alkyl(C₁-C₆)thio ; un radical amino ; un radical amino protégé par un radical alkyl(C₁-C₆)carbonyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
   - R₅ représente un radical alkyle en C₁-C₆ ; un radical monohydroxyalkyle en C₁-C₆ ; un radical polyhydroxyalkyle en C₂-C₆ ; un radical cyanoalkyle en C₁-C₆ ; un radical trialkyl(C₁-C₆)silanealkyle en C₁-C₆ ; un radical alcoxy(C₁-C₆)alkyle en C₁-C₆ ; un radical carbamylalkyle C₁-C₆ ; un radical alkyl(C₁-C₆)carboxyalkyle en C₁-C₆ ; un radical benzyle ;
   - R₆, R₇ et R₈, identiques ou différents, représentent un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical cyanoalkyle en C₁-C₆ ; un radical benzyle ; un radical amidoalkyle en C₁-C₆ ; un radical trialkyl(C₁-C₆)silanealkyle en C₁-C₆ ou un radical aminoalkyle en C₁-C₆ dont l'amine est protégée par un radical alkyl(C₁-C₆)carbonyle ou alkyl(C₁-C₆)sulfonyle ; deux des radicaux R₆, R₇ et R₈ peuvent également former ensemble, avec l'atome d'azote auquel ils sont rattachés, un cycle saturé à 5 ou 6 chaînons carboné ou contenant un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle ou alkyl(C₁-C₆)sulfonyle ;
   - R₉ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆ ; un radical aminoalkyle en C₁-C₆ dont l'amine est protégée par un radical alkyl(C₁-C₆)carbonyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)Sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆; un radical N-alkyl(C₁-C₆)sulfonamidoalkyle en C₁-C₆ ;
   - a et y sont des nombres entiers égaux à 0 ou 1 ; avec les conditions suivantes :
      - dans les groupements cationiques insaturés de formule (II) :
         - lorsque a = 0, le bras de liaison D est rattaché à l'atome d'azote,
         - lorsque a = 1, le bras de liaison D est rattaché à l'un des sommets E, G, J ou L,
         - y ne peut prendre la valeur 1 que :
            1) lorsque les sommets E, G, J et L représentent simultanément un atome de carbone, et que le radical R₅ est porté par l'atome d'azote du cycle insaturé ; ou bien
            2) lorsqu'au moins un des sommets E, G, J et L représente un atome d'azote sur lequel le radical R₅ est fixé ;
      - dans les groupements cationiques insaturés de formule (III) :
         - lorsque a = 0, le bras de liaison D est rattaché à l'atome d'azote,
         - lorsque a = 1, le bras de liaison D est rattaché à l'un des sommets E, G, J, L ou M,
         - y ne peut prendre la valeur 1 que lorsqu'au moins un des sommets E, G, J, L et M représente un atome divalent, et que le radical R₅ est porté par l'atome d'azote du cycle insaturé ;
      - dans les groupements cationiques de formule (IV) :
         - lorsque a = 0, alors le bras de liaison D est rattaché à l'atome d'azote portant les radicaux R₆ à R₈,
         - lorsque a = 1, alors deux des radicaux R₆ à R₈ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
   - X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
étant entendu que le nombre de groupement cationique Z est égal à 1.

Comme indiqué précédemment, les colorations obtenues avec la composition de teinture d'oxydation contenant un ou plusieurs composés de formule (I) conforme à l'invention sont puissantes et permettent d'atteindre une large palette de couleurs. Elles présentent de plus d'excellentes propriétés de résistance vis à vis de l'action des différents agents extérieurs (lumière, intempéries, lavage, ondulation permanente, transpiration, frottements). Ces propriétés sont particulièrement remarquables notamment en ce qui concerne la résistance des colorations obtenues vis à vis de l'action de la lumière, des lavages, de l'ondulation permanente et de la transpiration.

Dans la formule (I) ci-dessus les radicaux alkyle et alcoxy peuvent être linéaires ou ramifiés.

Parmi les cycles des groupements insaturés Z de formule (II) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Lorsque les composés de formule (I) sont tels qu'ils comportent un groupe OH sur l'une des positions 3 ou 5, en α d'un atome d'azote, il existe un équilibre tautomérique pouvant être représenté par exemple par le schéma suivant :

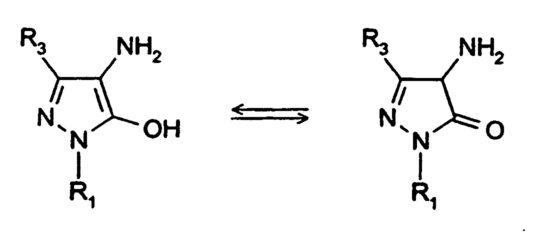

Parmi les composés de formule (I) ci-dessus, on peut notamment citer :
- le chlorure de [3-(4-amino-2,5-diméthyl-2H-pyrazol-3-ylamino)-propyl]-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-1-yl)-éthyl]-1-méthyl-3H-imidazol-1-ium ;
- le chlorure de 3-[2-(4,5-diamino-pyrazol-1-yl)-éthyl]-1-méthyl-3H-imidazol-1-ium;
- le chlorure de [2-(4,5-diamino-3-méthyl-pyrazol-1-yl)-éthyl]-triméthylammonium ;
- le chlorure de [2-(4,5-diamino-pyrazol-1-yl)-éthyl]-triméthyl-ammonium ;
- le chlorure de [2-(4-amino-5-hydroxy-pyrazol-1-yl)-éthyl]-triméthyl-ammonium ;
- le chlorure de [2-(4-amino-5-hydroxy-3-méthyl-pyrazol-1-yl)-éthyl]-triméthylammonium ;
- le chlorure de 3-[2-(4-amino-5-hydroxy-3-méthyl-pyrazol-1-yl)-éthyl]-1-méthyl-3H-imidazol-1-ium ;
- le chlorure de 3-[2-(4-amino-5-hydroxy-pyrazol-1-yl)-éthyl]-1-méthyl-3H-imidazol-1-ium ;
- le chlorure de 3-[2-(4,5-diamino-1-méthyl- H-pyrazol-3-yl)-éthyl]-1-méthyl-3H-imidazol-1-ium,
et leurs sels d'addition avec un acide ou avec une base, et leurs possibles formes tautomères.

Parmi ces composés de formule (I), on préfère plus particulièrement:
- le chlorure de [3-(4-amino-2,5-diméthyl-2H-pyrazol-3-ylamino)-propyl-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-1-yl)-éthyl]-1-méthyl-3H-imidazol-1-ium ;
- le chlorure de 3-[2-(4,5-diamino-1-méthyl-1H-pyrazol-3-yl)-éthyl]-1-méthyl-3H-imidazol-1-ium ;
et leurs sels d'addition avec un acide ou avec une base, et leurs possibles formes tautomères.

Les composés de formule (I) conformes à l'invention peuvent être facilement obtenus, selon des méthodes bien connues de l'état de la technique :
- soit par réduction des composés nitrés ou nitrosés cationiques correspondants. Dans ce cas, la réduction en amine aromatique primaire correspondante est effectuée selon des méthodes classiques (J. Lehmman dans "Houben-Weyl, "Methoden der Organischen Chemie", Band IV/1c : Reduktion I page 491 à 537, 1980). Les méthodes préférées selon l'invention font intervenir des métaux comme Zn, Sn, ou Fe en milieu acide comme l'acide chlorhydrique aqueux, ou l'acide acétique aqueux en présence ou non d'un co-solvant comme le méthanol, l'éthanol ou le tétrahydrofurane. L'hydrogénation catalytique est une méthode de réduction préférée selon l'invention. Cette hydrogénation catalytique utilise des métaux comme le palladium, le platine ou le nickel. On préfère encore plus particulièrement le palladium sur charbon ou le nickel de Raney, ou bien encore des oxydes comme PtO₂ dans des solvants comme le méthanol, l'éthanol, le tétrahydrofurane ou l'acétate d'éthyle en présence ou non d'un acide comme par exemple l'acide acétique. Ces réductions catalytiques peuvent aussi être effectuées avec de l'acide formique en présence d'une trialkylamine comme la triéthylamine ou avec du formiate d'ammonium à la place de l'hydrogène gazeux. (S. Ram, R.E. Ehrenkaufer, Synthesis,1988,91).
- soit par réduction des composés azoïques cationiques correspondants (coupure réductrice). La réduction en amine aromatique primaire correspondante est effectuée selon des méthodes classiques (J. Lehmman dans "Houben-Weyl, "Methoden der Organischen Chemie", Band IV/1c: Reduktion I page 551 à 553, 1980 ; E.C. Taylor & Coll., J. Amer. Chem. Soc, 80, 421, 1958).

Cette étape de réduction (obtention d'une amine aromatique primaire) qui confère au composé synthétisé son caractère de composé oxydable (de base d'oxydation) suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de benzènesulfonation, etc...), faire ensuite la ou les substitutions ou modifications désirées (y compris la quatemisation) et terminer par le "déprotection" (en général en milieu acide) de la fonction amine.

Lorsque la synthèse est terminée, les composés de formule (I) conformes à l'invention peuvent, le cas échéant, être récupérés par des méthodes bien connues de l'état de la technique telles que la cristallisation, la distillation.

Un autre objet de l'invention est l'utilisation des composés de formules (I) conformes à l'invention à titre de base d'oxydation pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de base d'oxydation, dans un milieu approprié pour la teinture, au moins un composé de formule (I) conforme à l'invention.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 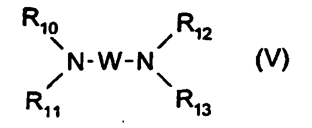 dans laquelle W est un reste propylène éventuellement substitué par un groupement hydroxyle ou un radical alkyle en C₁-C₆ ; R₁₀, R₁₁, R₁₂ et R₁₃, identiques ou différents, représentent un atome d'hydrogène, un radical alkyle en C₁-C₆ ou hydroxyalkyle en C₁-C₆.

La composition tinctoriale conforme à l'invention peut encore contenir, en plus du ou des composés de formule (I) définie ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques différentes des composés de formule (I).

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols, on peut plus particulièrement citer à titre d'exemple, citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques non cationiques

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs possibles formes tautomères, et leurs sels d'addition avec un acide.

Parmi les dérivés pyrazoliques non cationiques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyt pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, -le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre de l'invention (composés de formule (I), bases d'oxydation additionnelles et coupleurs) sont notamment choisis parmi les chlorhydrates, les bromhydrates, les sulfates, les citrates, les succinates, les tartrates, les lactates et les acétates. Les sels d'addition avec une base utilisables dans le cadre de l'invention (composés de formule (I)), sont notamment ceux obtenus avec la soude, la potasse, l'ammoniaque ou les amines.

La composition tinctoriale conforme l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES DE PREPARATION

### EXEMPLE DE PREPARATION 1 : Synthèse du chlorure de 3-[3-(4-amino. 2,5-diméthyl-2H-pyrazol-3-ylamino)-propyl]-1-(2-hydroxyéthyl)-3H-imidazol-1-ium, dichlorhydrate

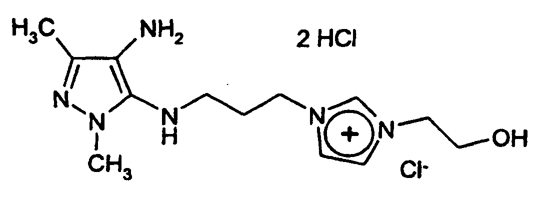

### a) Préparation de la (2,5-diméthyl-4-nitro-2H-pyrazol-3-yl)-(3-imidazol-1-yl-propyl)-amine

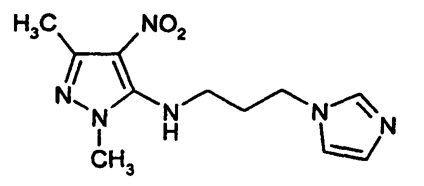

On a introduit, dans un ballon tricol de 25 cc, équipé d'une agitation magnétique, d'un thermomètre et d'un réfrigérant, 0,88 g de 5-chloro-1,3-diméthyl-4-nitro-1 H-pyrazole (Aldrich), 1 équivalent molaire de triéthylamine, 1,1 équivalent molaire de 3-imidazol-1-yl-propylamine et 5 cc de N,N-diméthylformamide. On a porté le milieu réactionnel à une température d'environ 105°C pendant 6 heures. On a évaporé le solvant sous vide. On a obtenu un liquide noir qu'on a purifié par chromatographie sur gel de silice (acétate d'éthyle/méthanol = 4/1). On a obtenu 0,75 g de (2,5-diméthyl-4-nitro-2H-pyrazol-3-yl)-(3-imidazot-1-yl-propyl)-amine sous forme de cristaux blancs avec une rendement de 56,7%.

### b) Préparation du chlorure de 3-[3-(2,5-diméthyl-4-nitro-2H-pyrazol-3-ylamino)-propyl]-1-(2-hydroxyéthyl)-3H-imidazol-1-ium

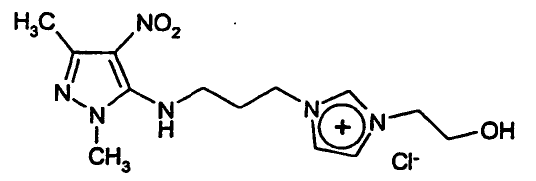

On a introduit, dans un ballon tricol de 10 cc, équipé d'une agitation magnétique, d'un thermomètre et d'un réfrigérant, 0,57 g de (2,5-diméthyl-4-nitro-2H-pyrazol-3-yl)-(3-imidazol-1-yl-propyl)-amine et 2,2 g de 2-chloroéthanol. On a porté le milieu réactionnel au reflux pendant 2 heures. On a évaporé le solvant sous vide. On a obtenu 0,7 g de chlorure de 3-[3-(2,5-diméthyl-4-nitro-2H-pyrazol-3-ylamino)-propyl]-1-(2-hydroxyéthyl)-3H-imidazol-1-ium sous forme d'un liquide visqueux avec un rendement de 96%.

### c) Préparation du chlorure de 3-[3-(4-amino-2,5-diméthyl-2H-pyrazol-3-ylamino)-propyl]-1-(2-hydroxyéthyl)-3H-imidazol-1-ium dichlorhydrate

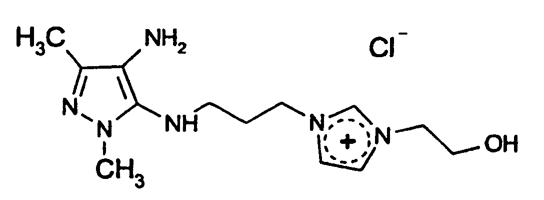

On a introduit dans un réacteur à hydrogénation de 500 cc, 7,9 g de chlorure de 3-[3-(2,5-diméthyl-4-nitro-2H-pyrazol-3-ylamino)propyl]-1-(2-hydroxyéthyl)-3H-imidazol-1-ium obtenu ci-dessus à l'étape précédente, 150 cc de méthanol et 0,97 g de palladium sur charbon à 5% contenant environ 50% d'eau. On a mis une pression d'hydrogène de 10 bars et porté le milieu réactionnel à 115°C. Après 5 heures, on a filtré le catalyseur sur célite sur 100 cc d'une solution d'éthanol chlorhydrique à 5 moles/litre. On a évaporé le solvant sous vide à la pompe à palette (0,1 bar). On a obtenu 6,5 g de chlorure de 3-[3-(4-amino-2,5-diméthyl-2H-pyrazol-3-ylamino)-propyl)-1-(2-hydroxyéthyl)-3H-imidazol-1-ium dichlorhydrate sous forme d'un solide avec un rendement de 73%.

L'analyse RMN ¹H (DMSO d6 + CD₃OD) était la suivante :
2,03 (m ; 2H) ; 2,13 (s ; 3H) ; 3,03 (t ; 2H) ; 3,57 (s ; 3H) ; 3,72 (t ; 2H) ; 4,24 (t ; 2H) ; 4,34 (t ; 2H) ; 7,73 (dd ; 1 H) ; 9,33 (s ; 1H).

### EXEMPLE DE PREPARATION 2 : Synthèse du chlorure de [3-(4-amino-2,5-diméthyl-2H-pyrazol-3-ylamino)-propyl]-(2-hydroxyéthyl)-diméthyl-ammonium, dichlorhydrate

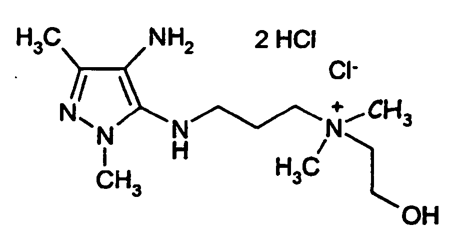

### a) Préparation du chlorure de [3-(2,5-diméthyl-4-nitro-2H-pyrazol-3-ylamino)-propyl]-(2-hydroxyéthyl)-diméthyl ammonium

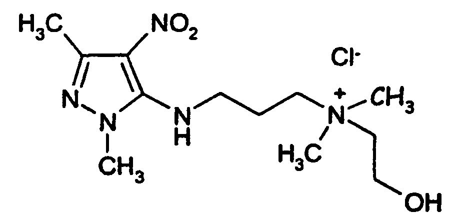

On a introduit, dans un ballon tricol de 10 cc, équipé d'une agitation magnétique, d'un thermomètre et d'un réfrigérant, 0,5 g de N-(2,5-diméthyl-4-nitro-2H-pyrazol-3-yl)-N',N'-diméthyl-propane-1,3-diamine et 2,2 g de 2-chloroéthanol. On a porté le milieu réactionnel au reflux pendant 2 heures. On a refroidit le milieu à température ambiante et on l'a dilué avec 50 cc d'acétate d'éthyle. On a filtré le précipité. On a obtenu 0,55 g de chlorure de [3-(2,5-diméthyl-4-nitro-2H-pyrazol-3-ylamino)-propyl-(2-hydroxyéthyl)-diméthyl ammonium sous forme de cristaux jaunes avec un rendement de 82%.

### b) Préparation du chlorure de [3-(4-amino-2,5-diméthyl-2H-pyrazol-3-ylamino)-propyl]-(2-hydroxyéthyl)-diméthyl-ammonium, dichlorhydrate

On a introduit, dans un réacteur à hydrogénation de 250 cc, 3,22 g de chlorure de [3-(2,5-diméthyl-4-nitro-2H-pyrazol-3-ylamino)-propyil]-(2-hydroxyéthyl)-diméthyl ammonium, 150 cc de méthanol et 0,42 g de palladium sur charbon à 5% contenant environ 50% d'eau. On a soumis le réacteur à une pression d'hydrogène de 11,7 bars et on a porté le milieu réactionnel à 60°C. Après 2 heures, la pression d'hydrogène était de 8,3 bars. On a filtré le catalyseur sur célite. On a fait passer un courant d'acide chlorhydrique gazeux à travers le filtrat et évaporé le solvant sous vide. On a obtenu 3 g de liquide visqueux qu'on a dilué dans 100 cc d'eau et qu'on a ensuite lyophilisé. On a obtenu 1,54 g de chlorure de [3-(4-amino-2,5-diméthyl-2H-pyrazol-3-ylamino)-propyl]-(2-hydroxyéthyl)-diméthyl-ammonium, dichlorhydrate, (contenant 1,3 moles d'eau), sous forme d'un solide avec un rendement de 40%, et dont l'analyse élémentaire calculée pour C₁₂ H₂₆ N₅ O . Cl , 2 HCl , 1,3 H₂O, (PM=388,15 g/mole), était la suivante :

| | % | C | H | N | O | Cl |
|---|---|---|---|---|---|---|
| Calculé | | 37,09 | 7,88 | 18,03 | 9,48 | 27,43 |
| Trouvé | | 37,46 | 7,88 | 17,79 | 9,68 | 26,85 |

### EXEMPLES D'APPLICATION

### EXEMPLES 1 à 8 DE TEINTURE EN MILIEU BASIQUE

On a préparé les compositions tinctoriales suivantes (teneurs en grammes) :

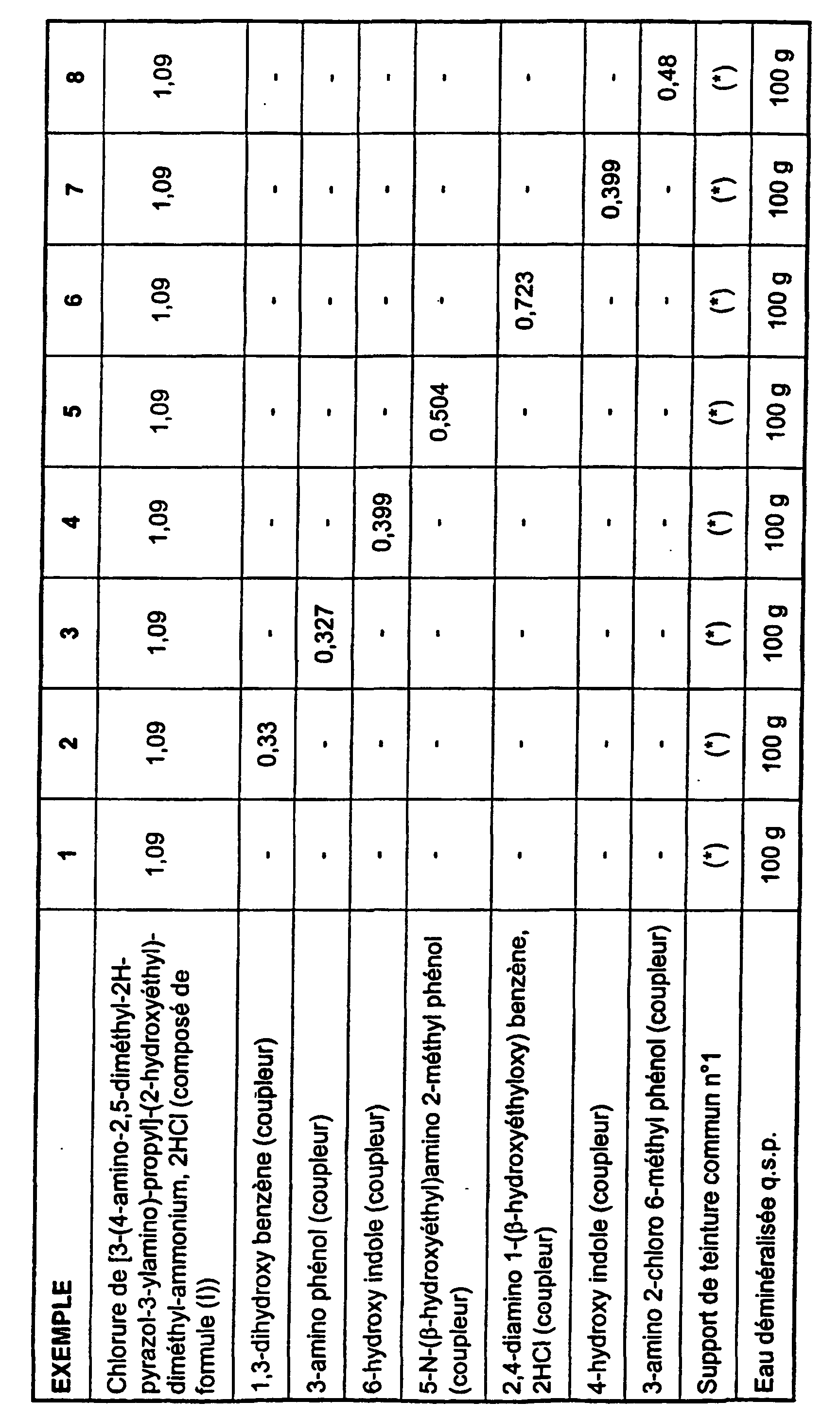

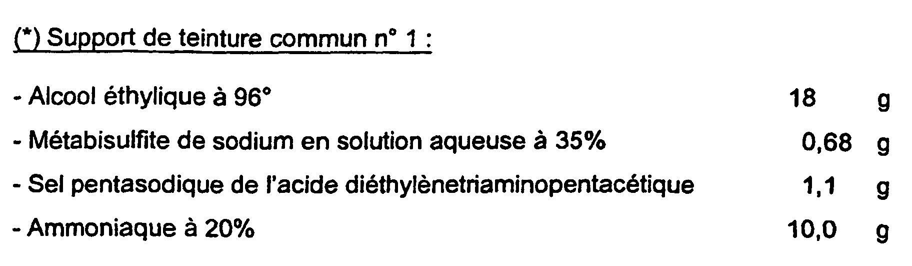

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris permanentés à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **1** | 10 ± 0,2 | Violine cendré léger |
| **2** | 10 ± 0,2 | Blond clair cendré doré légèrement irisé |
| **3** | 10 ± 0,2 | Violine cendré |
| **4** | 10 ± 0,2 | Blond foncé marron cendré |
| **5** | 10 ± 0,2 | Châtain clair irisé violacé |
| **6** | 10 ± 0,2 | Bleu vert rabattu |
| **7** | 10 ± 0,2 | Châtain cendré violacé |
| **8** | 10 ± 0,2 | Violine |

### EXEMPLES 9 à 12 DE TEINTURE EN MILIEU NEUTRE

On a préparé les compositions tinctoriales suivantes (teneurs en grammes) :

| **EXEMPLE** | **9** | **10** | **11** | **12** |
|---|---|---|---|---|
| Chlorure de [3-(4-amino-2,5-diméthyl-2H-pyrazol-3-ylamino)-propyl]-(2-hydroxy-éthyl)-diméthyl-ammonium, 2HCl (composé de formule (I)) | 1,09. | 1,09 | 1,09 | 1,09 |
| 5-N-(β-hydroxyéthyl)amino 2-méthyl phénol (coupleur) | 0,504 | - | - | - |
| 2,4-diamino 1-(β-hydroxyéthyloxy) benzène, 2HCl (coupleur) | - | 0,723 | - | - |
| 6-hydroxy indole (coupleur) | - | - | 0,399 | - |
| 4-hydroxy indole (coupleur) | - | - | - | 0,399 |
| Support de teinture commun n°2 . | (**) | (**) | (**) | (**) |
| Eau déminéralisée q.s.p. | 100 g | 100 g | 100 g | 100 g |

| | | | | |
|---|---|---|---|---|
| (**) Support de teinture commun n° 2 : - Ethanol à 96° 18 g - Tampon K₂HPO₄ /KH₂PO₄ (1,5 M / 1M) 10 g - Métabisulfite de sodium 0,68 g - Sel pentasodique de l'acide diéthylènetriaminopentacétique 1,1 g | | | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **9** | 5,7 ± 0,2 | Violine cendré |
| **10** | 5,7 ± 0,2 | Vert |
| **11** | 5,7 ± 0,2 | Blond clair légèrement violacé |
| **12** | 5,7 ± 0,2 | Violine rouge léger |

## Revendications

1. Composés de formule (I) suivante, leurs sels d'addition avec un acide ou avec une base, et leurs possibles formes tautomères : 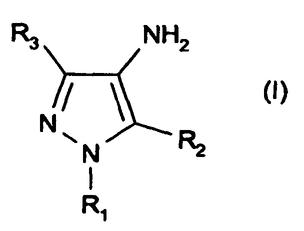 dans laquelle :
• R₁ représente un atome d'hydrogène ; un groupement Z tel que défini ci-après ; un radical alkyle en C₁-C₆ ; un radical trifluoroalkyle en C₁-C₆ ; un radical monohydroxyalkyle en C₁-C₆ ; un radical polyhydroxyalkyle en C₂-C₆ ; un radical arylalkyle en C₁-C₆ dans lequel le radical aryle est un radical phényle ou un hétérocycle aromatique à cinq ou six chaînons : un radical alcoxy(C₁-C₆)alkyle en C₁-C₆ ; un radical alkyl(C₁-C₆)thio alkyle en C₁-C₆; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical aminosulfonylalkyle(C₁-C₆) ; un radical N-alkyl(C₁-C₆) aminosulfonylalkyle(C₁-C₆) ; un radical N,N-dialkyl(C₁-C₆) aminosulfonylalkyle(C₁-C₆) ;
• R₂ et R₃, identiques ou différents représentent un groupement -NHR₄ ; un radical hydroxyle ; un atome d'halogène ; un radical nitro, un radical cyano ; un radical carboxy ; un radical carboxy alkyle en C₁-C₆ ; un radical carbamyle ; un radical N-alkyl(C₁-C₆)carbamyle; un radical N,N-dialkyl(C₁-C₆)carbamyle; un radical alkoxy en C₁-C₆ ; un radical thioalkyle en C₁-C₆ ou l'une des significations données ci-dessus pour R₁ ; étant entendu qu'au moins un des radicaux R₂ et R₃ représente un groupement -NHR₄ ou un radical hydroxyle;
• R₄ représente un atome d'hydrogène ; un groupement Z tel que défini ci-après ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, alkyl(C₁-C₆)sulfonyle, formyle, trifluoroalkyl(C₁-C₆)carbonyle; l'amine du radical aminoalkyle en C₁-C₆ peut également être substituée par deux radicaux formant, conjointement avec l'atome d'azote de ladite amine, un cycle saturé ou insaturé à cinq ou six chaînons et pouvant contenir un ou plusieurs hétéroatomes choisis parmi l'azote, l'oxygène et le soufre ;
• Z est choisi parmi les groupements cationiques insaturés de formules (II) et (III) suivantes, et les groupements cationiques saturés de formule (IV) suivante : 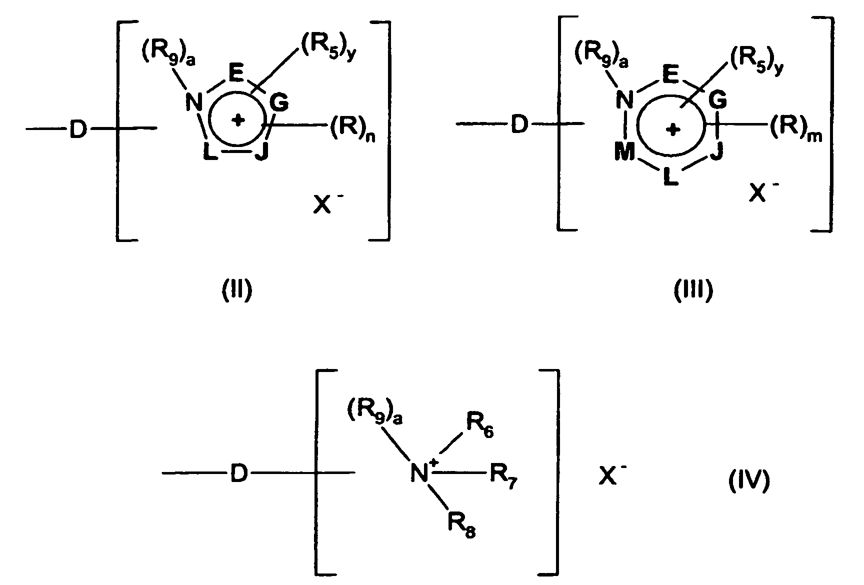 dans lesquelles :
• D est un bras de liaison qui représente une chaîne alkyle comportant de préférence de 1 à 14 atomes de carbone, linéaire ou ramifiée pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone ou d'azote ;
• n est un nombre entier compris entre 0 et 4 inclusivement ;
• m est un nombre entier compris entre 0 et 5 inclusivement ;
• les radicaux R, identiques ou différents, représentent un atome d'halogène ; un radical hydroxyle ; un radical alkyle en C₁-C₆ ; un radical monohydroxyalkyle en C₁-C₆ ; un radical polyhydroxyalkyle en C₂-C₆ ; un radical nitro ; un radical cyano ; un radical cyanoalkyle en C₁-C₆ ; un radical alcoxy en C₁-C₆ ; un radical trialkyl(C₁-C₆)silanealkyle en C₁-C₆ ; un radical amido ; un radical aldéhydo ; un radical carboxyle ; un radical alkylcarbonyle en C₁-C₆ ; un radical thio ; un radical thioalkyle en C₁-C₆ ; un radical alkyl(C₁-C₆)thio ; un radical amino ; un radical amino protégé par un radical alkyl(C₁-C₆)carbonyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₅ représente un radical alkyle en C₁-C₆ ; un radical monohydroxyalkyle en C₁-C₆ ; un radical polyhydroxyalkyle en C₂-C₆ ; un radical cyanoalkyle en C₁-C₆ ; un radical trialkyl(C₁-C₆)silanealkyle en C₁-C₆ ; un radical alcoxy(C₁-C₆)alkyle en C₁-C₆ ; un radical carbamylalkyle C₁-C₆ ; un radical alkyl(C₁-C₆)carboxyalkyle en C₁-C₆ ; un radical benzyle ;
• R₆, R₇ et R₈, identiques ou différents, représentent un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆; un radical cyanoalkyle en C₁-C₆ ; un radical benzyle ; un radical amidoalkyle en C₁-C₆ ; un radical trialkyl(C₁-C₆)silanealkyle en C₁-C₆ ou un radical aminoalkyle en C₁-C₆ dont l'amine est protégée par un radical alkyl(C₁-C₆)carbonyle ou alkyl(C₁-C₆)sulfonyle; deux des radicaux R₆, R₇ et R₈ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle ou alkyl(C₁-C₆)sulfonyle ;
• R₉ représente un radical alkyle en C₁-C₆ ; un radical monohydroxyalkyle en C₁-C₆ ; un radical polyhydroxyalkyle en C₂-C₆ ; un radical benzyle ; un radical aminoalkyle en C₁-C₆ ; un radical aminoalkyle en C₁-C₆ dont l'amine est protégée par un radical alkyl(C₁-C₆)carbonyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ un radical N-alkyl(C₁-C₆)carbamylalkyle en C₁-C₆ ; un radical N-alkyl(C₁-C₆)sulfonamidoalkyle en C₁-C₆ ;
• a et y sont des nombres entiers égaux à 0 ou 1 ; avec les conditions suivantes :
- dans les groupements cationiques insaturés de formule (II) :
- lorsque a = 0, le bras de liaison D est rattaché à l'atome d'azote,
- lorsque a = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₅ est porté par l'atome d'azote du cycle insaturé ; ou bien
2) lorsqu'au moins un des sommets E, G, J et L représente un atome d'azote sur lequel le radical R₅ est fixé ;
- dans les groupements cationiques insaturés de formule (III) :
- lorsque a = 0, le bras de liaison D est rattaché à l'atome d'azote,
- lorsque a = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₅ est porté par l'atome d'azote du cycle insaturé ;
- dans les groupements cationiques de formule (IV) :
- lorsque a = 0, alors le bras de liaison D est rattaché à l'atome d'azote portant les radicaux R₆ à R₈,
- lorsque a = 1, alors deux des radicaux R₆ à R₈ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
• X⁻ représente un anion monovalent ou divalent ;
étant entendu que le nombre de groupement cationique Z est égal à 1.

2. Composés selon la revendication 1, **caractérisés par le fait que** les cycles des groupements insaturés Z de formule (II) sont choisis parmi les cycles pyrrolique, imidazolique, pyrazolique, et triazolique.

3. Composés selon la revendication 1, **caractérisés par le fait que** les cycles des groupements insaturés Z de formule (III) sont choisis parmi les cycles pyridinique, pyrimidinique, pyrazinique, et triazinique.

4. Composés selon l'une quelconque des revendications précédentes, **caractérisés par le fait que** deux des radicaux R₆, R₇ et R₈ forment un cycle pyrrolidine, un cycle pipéridine, un cycle pipérazine ou un cycle morpholine.

5. Composés selon l'une quelconque des revendications précédentes, **caractérisés par le fait que** X⁻ est choisi parmi un atome d'halogène, un hydroxyde, un hydrogènesulfate, ou un alkyl(C₁-C₆)sulfate.

6. Composés selon l'une quelconque des revendications précédentes, **caractérisés par le fait qu'**ils sont choisis parmi :
- le chlorure de [3-(4-amino-2,5-diméthyl-2H-pyrazol-3-ylamino)-propyl]-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-1-yl)-éthyl]-l-méthyl-3H-imidazol-1-ium ;
- le chlorure de -3-[2-(4,5-diamino-pyrazol-1-yl)-éthyl]-1-méthyl-3H-imidazol 1-ium;
- le chlorure de [2-(4,5-diamino-3-méthyl-pyrazol-1-yl)-éthyl]-triméthyl-ammonium ;
- le chlorure de [2-(4,5-diamino-pyrazol-1-yl)-éthyl]-triméthyl-ammonium ;
- le chlorure de [2-(4-amino-5-hydroxy-pyrazol-1-yl)-éthyl]-triméthyl-ammonium ;
- le chlorure de [2-(4-amino-5-hydroxy-3-méthyl-pyrazol-1-yl)-éthyl]-triméthyl-ammonium ;
- le chlorure de 3-[2-(4-amino-5-hydroxy-3-méthyl-pyrazol-1-yl)-éthyl]-1-méthyl-3H-imidazol-1-ium;
- le chlorure de 3-[2-(4-amino-5-hydroxy-pyrazol-1-yl)-éthyl]-1-méthyl-3H-imidazol-1-ium ;
- le chlorure de 3-[2-(4,5-diamino-1-méthyl-1H-pyrazol-3-yl)-éthyl]-1-méthyl-3H-imidazol-1-ium,
et leurs sels d'addition avec un acide ou avec une base, et leurs possibles formes tautomères.

7. Composés selon la revendication 6, **caractérisés par le fait qu'**ils sont choisis parmi:
- le chlorure de [3-(4-amino-2,5-diméthyl-2H-pyrazol-3-ylamino)-propyl]-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-yl)-éthyl]-1-méthyl-3H-imidazol-1-ium ;
- le chlorure de 3-[2-(4,5-diamino-1-méthyl-1H-pyrazol-3-yl)-éthyl]-1-méthyl-3H-imidazol-1-ium ;
et leurs sels d'addition avec un acide ou avec une base, et leurs possibles formes tautomères.

8. Composés selon l'une quelconque des revendications précédentes, **caractérisés par le fait que** les sels d'addition avec un acide sont choisis parmi les chlorhydrates, les bromhydrates, les sulfates, les citrates, les succinates, les tartrates, les lactates et les acétates et que les sels d'addition avec une base sont choisis parmi ceux obtenus avec la soude, la potasse, l'ammoniaque ou les amines.

9. Utilisation des composés de formule. (I) tels que définis à l'une quelconque des revendications 1 à 8, à titre de base d'oxydation pour la teinture d'oxydation des fibres kératiniques.

10. Composition pour la teinture d'oxydation des fibres kératiniques, **caractérisée par le fait qu'**elle comprend à titre de base d'oxydation, dans un milieu approprié pour la teinture, au moins un composé de formule (I) tel que défini à l'une quelconque des revendications 1 à 8.

11. Composition selon la revendication 10, **caractérisée par le fait que** le ou les composés de formule (I) représentent de 0,0005 à 12 % en poids du poids total de la composition tinctoriale.

12. Composition selon la revendication 11, **caractérisée par le fait que** le ou les composés de formule (I) représentent de 0,005 à 6 % en poids du poids total de la composition tinctoriale.

13. Composition selon l'une quelconque des revendications 10 à 12, **caractérisée par le fait qu'**elle renferme au moins une base d'oxydation additionnelle choisie parmi les paraphénylènediamines, les bis-phénylalkylènediamines, les para-aminophénols, les ortho-aminophénols et les bases hétérocycliques différentes des composés de formule (1).

14. Composition selon la revendication 13, **caractérisée par le fait que** la ou les bases d'oxydation additionnelles représentent de 0,0005 à 12 % en poids du poids total de la composition tinctoriale.

15. Composition selon l'une quelconque des revendications 10 à 14, **caractérisée par le fait qu'**elle renferme au moins un coupleur et/ou au moins un colorant direct.

16. Composition selon la revendication 15, **caractérisée par le fait que** le ou les coupleurs sont choisis parmi les métaphénylènediamines, les méta-aminophénols, les métadiphénols et les coupleurs hétérocycliques, et leurs sels d'addition avec un acide.

17. Composition selon la revendication 16, **caractérisée par le fait que** le ou les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

18. Composition selon l'une quelconque des revendications 15 à 17, **caractérisée par le fait que** le ou les coupleurs représentent de 0,0001 à 10 % en poids du poids total de la composition tinctoriale.

19. Composition selon l'une quelconque des revendications 10 à 18, **caractérisée par le fait que** les sels d'addition avec un acide sont choisis parmi les chlorhydrates, les bromhydrates, les sulfates, les citrates, les succinates, les tartrates, les lactates et les acétates.

20. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 10 à 19, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

21. Procédé selon la revendication 20, **caractérisé par le fait que** l'agent oxydant est choisi parmi le peroxyde d'hydrogène, le peroxyde d'urée, les bromates de métaux alcalins, les persels tels que les perborates et persulfates et les enzymes.

22. Dispositif à plusieurs compartiments, ou "kit" de teinture à plusieurs compartiments, dont un premier compartiment renferme une composition tinctoriale telle que définie à l'une quelconque des revendications 10 à19 et un second compartiment renferme une composition oxydante.

## Patentansprüche

1. Verbindungen der folgenden Formel (I) und ihre Additionssalze mit einer Säure oder einer Base und ihre möglichen tautomeren Formen. 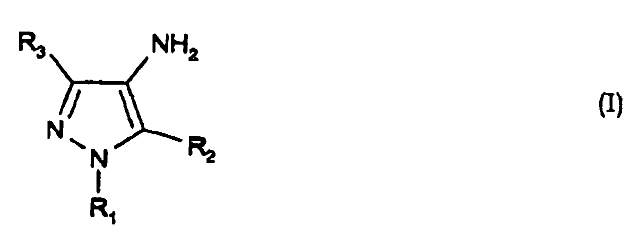 worin bedeuten:
· R₁ ein Wasserstoffatom; eine nachstehend definierte Gruppe Z; C₁-₆-Alkyl; C₁₋₆-Trifluoralkyl; C₁₋₆-Monohydroxyalkyl; C₂₋₆-Polyhydroxyalkyl; Arylalkyl(C₁₋₆), wobei die Arylgruppe Phenyl oder einen 5- oder 6-gliedrigen, aromatischen Heterocyclus bedeutet; Alkoxy(C₁₋₆)alkyl(C₁₋₆); Alkyl(C₁₋₆)thioalkyl(C₁₋₆); Aminoalkyl(C₁₋₆)-carbonylalkyl(C₁₋₆); N-Alkyl(C₁₋₆)aminoalkyl(C₁₋₆)carbonylalkyl(C₁₋₆); N,N-Dialkyl(C₁₋₆)aminoalkyl(C₁₋₆)carbonylalkyl(C₁₋₆); Aminosulfonylalkyl (C₁₋₆); N-Alkyl(C₁₋₆)aminosulfonylalkyl(C₁₋₆); N,N-Dialkyl(C₁-₆)aminosulfonylalkyl(C₁₋₆);
· R₂ und R₃, die identisch oder voneinander verschieden sind, -NHR₄; Hydroxy; ein Halogenatom; Nitro; Cyano; Carboxy; Carboxyalkyl(C₁₋₆); Carbamoyl; N-Alkyl(C₁₋₆)carbamoyl; N,N-Dialkyl(C₁₋₆)-carbamoyl; C₁₋₆-Alkoxy; C₁₋₆-Thioalkyl; oder eine der oben für R₁ angegebenen Bedeutungen; mit der Maßgabe, dass mindestens eine der Gruppen R₂ und R₃ eine Gruppe NHR₄ oder Hydroxy bedeutet;
· R₄ ein Wasserstoffatom; eine nachstehend definierte Gruppe Z; C₁₋₆-Alkyl; C₁₋₆-Monohydroxyalkyl; C₂₋₆-Polyhydroxyalkyl; Alkoxy(C₁₋₆)alkyl(C₁₋₆); Benzyl; C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)carbamoylalkyl(C₁₋₆); C₁₋₆-Thiocarbamoylalkyl; C₁₋₆-Trifluoralkyl; C₁₋₆-Sulfoalkyl; Alkyl(C₁₋₆)carboxyalkyl(C₁₋₆); Alkyl(C₁₋₆)sulfinylalkyl(C₁₋₆); Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); C₁₋₆-Aminoalkyl ; C₁₋₆-Aminoalkyl, wobei die Aminogruppe mit einer oder zwei Gruppen substituiert ist, die gleich oder verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Alkyl(C₁₋₆)-sulfonyl, Formyl, Trifluoralkyl(C₁₋₆)carbonyl, wobei die Aminogruppe der C₁₋₆-Aminoalkylgruppe auch mit zwei Gruppen substituiert sein kann, die gemeinsam mit dem Stickstoffatom des Amins einen gesättigten oder ungesättigten, 5- oder 6-gliedrigen Ring bilden können, der ein oder mehrere Heteroatome enthalten kann, die unter Stickstoff, Sauerstoff und Schwefel ausgewählt sind;
· Z ist unter den ungesättigten kationischen Gruppen der folgenden Formeln (II) und (III) und den gesättigten kationischen Gruppen der folgenden Formel (IV) ausgewählt: 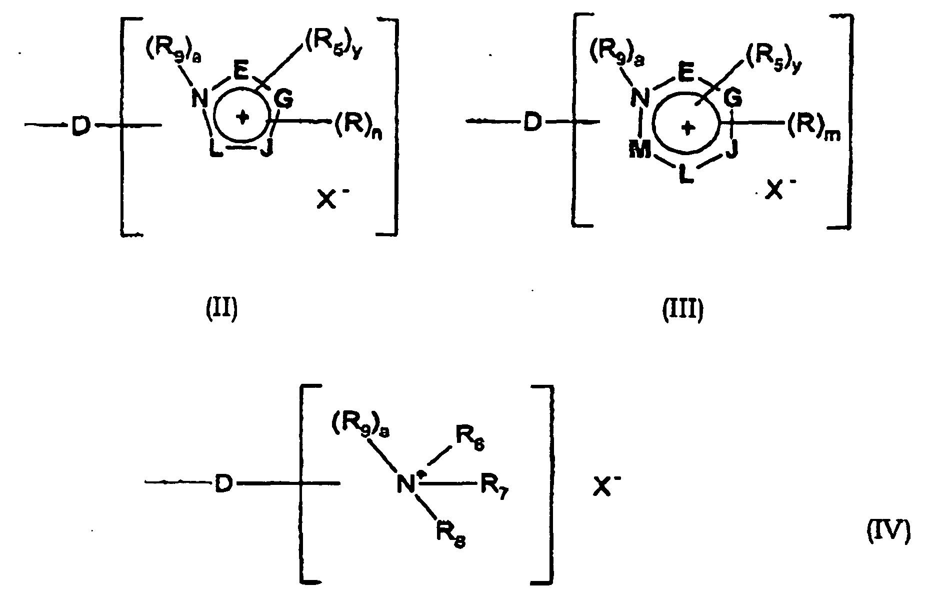 worin:
- D eine Verbindungsgruppe ist, die eine Alkylkette mit vorzugsweise 1 bis 14 Kohlenstoffatomen bedeutet, die geradkettig oder verzweigt vorliegt, mit einer oder mehreren Hydroxygruppen oder C₁₋₆-Alkoxygruppen substituiert sein kann und eine oder mehrere Ketofunktionen tragen kann;
- die Ringbestandteile E, G, J, L und M, die identisch oder voneinander verschieden sind, ein Kohlenstoffatom oder Stickstoffatom bedeuten;
- n 0 oder eine ganze Zahl von 1 bis 4 ist;
- m 0 oder eine ganze Zahl von 1 bis 5 ist;
- die Gruppen R, die identisch oder voneinander verschieden sind, Halogen; Hydroxy; C₁₋₆-Alkyl; C₁₋₆-Monohydroxyalkyl; C₂₋₆-Polyhydroxyalkyl; Nitro; Cyano; C₁₋₆-Cyanoalkyl; C₁₋₆-Alkoxy; Trialkyl(C₁₋₆)silanalkyl(C₁₋₆); Amido; Aldehydo; Carboxy; C₁₋₆-Alkylcarbonyl; Thio; C₁₋₆-Thioalkyl; Alkyl(C₁₋₆)thio; Amino; eine Aminogruppe, die mit Alkyl(C₁₋₆)carbonyl oder Alkyl(C₁₋₆)sulfonyl geschützt ist; oder eine Gruppe NHR" oder NR"R"' bedeuten, worin R" und R"', die identisch oder voneinander verschieden sind, C₁₋₆-Alkyl, C₁₋₆-Monohydroxyalkyl oder C₂₋₆-Polyhydroxyalkyl bedeuten;
- R₅ C₁₋₆-Alkyl; C₁₋₆-Monohydroxyalkyl; C₂₋₆-Polyhydroxyalkyl; C₁₋₆-Cyanoalkyl; Trialkyl(C₁₋₆)silanalkyl(C₁₋₆); Alkoxy(C₁₋₆)-alkyl(C₁₋₆); C₁₋₆-Carbamoylalkyl; Alkyl(C₁₋₆)carboxyalkyl(C₁₋₆) oder Benzyl bedeutet;
- R₆, R₇ oder R₈, die gleich oder verschieden sind, bedeuten: C₁₋₆-Alkyl; C₁₋₆-Monohydroxyalkyl; C₂₋₆-Polyhydroxyalkyl; Alkoxy(C₁₋₆)alkyl(C₁₋₆); C₁₋₆-Cyanoalkyl; Benzyl; C₁₋₆-Amidoalkyl; Trialkyl(C₁₋₆)silanalkyl(C₁₋₆) oder C₁₋₆-Aminoalkyl, wobei die Aminogruppe mit Alkyl(C₁₋₆)carbonyl oder Alkyl(C₁₋₆)sulfonyl geschützt ist; und zwei der Gruppen R₆, R₇ und R₈ auch gemeinsam mit dem Stickstoffatom, an das sie gebunden sind, einen 5-oder 6-gliedrigen gesättigten Ring bilden können, der auf Kohlenstoffatomen basiert oder ein oder mehrere Heteroatome enthält, wobei der Ring unsubstituiert vorliegen kann oder substituiert sein kann mit Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Ketoalkyl, Thio, C₁₋₆-Thioalkyl, Alkyl(C₁₋₆)thio, Amino, Amino, das mit Alkyl(C₁₋₆)carbonyl oder Alkyl(C₁₋₆)sulfonyl geschützt ist;
- R₉ C₁₋₆-Alkyl; C₁₋₆-Monohydroxyalkyl; C₂₋₆-Polyhydroxyalkyl; Benzyl; C₁₋₆-Aminoalkyl; C₁₋₆-Aminoalkyl, wobei die Aminogruppe mit Alkyl(C₁₋₆)carbonyl oder Alkyl(C₁₋₆₎sulfonyl geschützt ist; C₁₋₆-Carboxyalkyl; C₁₋₆-Cyanoalkyl; C₁₋₆-Carbamoylalkyl; C₁₋₆-Trifluoralkyl; Trialkyl(C₁₋₆)silanalkyl(C₁₋₆); C₁₋₆-Sulfonamidoalkyl; Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N-Alkyl(C₁-₆)carbamoylalkyl(C₁₋₆); N-Alkyl(C₁₋₆)sulfonamidoalkyl(C₁₋₆);
- a und y 0 oder 1 bedeuten; mit den folgenden Maßgaben:
in den ungesättigten kationischen Gruppen der Formel (II):
- die Verbindungsgruppe D ist an das Stickstoffatom gebunden, wenn a = 0.
- die Verbindungsgruppe D ist an eines der Atome E, G, J oder L gebunden, wenn a = 1,
- y kann nur den Wert 1 annehmen, wenn:
1) die Atome E, G, J und L gleichzeitig ein Kohlenstoffatom bedeuten und die Gruppe R₅ von dem Stickstoffatom des ungesättigten Rings getragen wird; oder
2) mindestens eines der Atome E, G, J und L ein Stickstoffatom bedeutet, an das R₅ gebunden ist;
in den ungesättigten kationischen Gruppen der Formel (III):
- die Verbindungsgruppe D an das Stickstoffatom gebunden ist, wenn a = 0,
- die Verbindungsgruppe D an eines der Atome E, G, J, L oder M gebunden ist, wenn a = 1,
- y nur den Wert 1 annehmen kann, wenn mindestens eines der Atome E, G, J, L und M ein zweiwertiges Atom bedeutet und die Gruppe R₅ von dem Stickstoffatom des ungesättigten Rings getragen wird;
in den kationischen Gruppen der Formel (IV):
- die Verbindungsgruppe D an das Stickstoffatom gebunden ist, das die Gruppen R₆ bis R₈ trägt, wenn a = 0,
- zwei der Gruppen R₆ bis R₈ gemeinsam mit dem Stickstoffatom, an das sie gebunden sind, einen oben definierten gesättigten 5- oder 6-gliedrigen Ring bilden und die Verbindungsgruppe D von einem Kohlenstoffatom des gesättigten Rings getragen wird, wenn a = 1;
- X⁻ ein einwertiges oder zweiwertiges Anion bedeutet;
mit der Maßgabe, dass:
- die Anzahl der kationischen Gruppen Z mindestens 1 ist.

2. Verbindungen nach Anspruch 1, **dadurch gekennzeichnet, dass** die Ringe der ungesättigten Gruppen Z der Formel (II) unter einem Pyrrolring, Imidazolring, Pyrazolring oder Triazolring ausgewählt sind.

3. Verbindungen nach Anspruch 1, **dadurch gekennzeichnet, dass** die Ringe der ungesättigten Gruppen Z der Formel (III) unter einem Pyridinring, Pyrimidinring, Pyrazinring oder Triazinring ausgewählt sind.

4. Verbindungen nach einem der vorhergehenden Ansprüche, **dadurch gekennzeichnet, dass** zwei der Gruppen R₆, R₇ und R₈ einen Pyrrolidinring, Piperidinring, Fiperazinring oder Morpholinring bilden.

5. Verbindungen nach einem der vorhergehenden Ansprüche, **dadurch gekennzeichnet, dass** X⁻ unter Halogen, Hydroxid, Hydrogensulfat oder Alkyl(C₁₋₆)sulfat ausgewählt ist.

6. Verbindungen nach einem der vorhergehenden Ansprüche, **dadurch gekennzeichnet, dass** sie ausgewählt sind unter:
- [3-(4-Amino-2,5-dimethyl-2H-pyrazol-3-ylamino)-propyl]-trimethyl-ammoniumchlorid;
- [3-(4-Amino-2,5-dimethyl-2H-pyrazol-3-ylamino)-propyl]-(2-hydroxy-ethyl)-dimethyl-ammoniumchlorid;
- 3-[3-(4-Amino-2,5-dimethyl-2H-pyrazol-3-ylamino)-propyl]-1-(2-hydroxy-ethyl)-3H-imidazol-1-ium-chlorid;
- 3-[(4-Amino-2H-pyrazol-3-ylcarbamoyl)-methyl]-1-methyl-3H-imidazol-1-ium-chlorid;
- 3-[2-(4,5-Diamino-3-methyl-pyrazol-1-yl)-ethyl]-1-methyl-3H-imidazol-1-ium-chlorid;
- 3-[2-(4,5-Diamino-pyrazol-1-yl)-ethyl]-1-methyl-3H-imidazol-1-ium-chlorid;
- [2-(4,5-Diamino-3-methyl-pyrazol-1-yl)-ethyl]-trimethylammonium-chlorid;
- [2-(4,5-Diamino-pyrazol-1-yl)-ethyl]-trimethyl-ammoniumchlorid;
- [2-(4-Amino-5-hydroxy-pyrazol-1-yl)-ethyl]-trimethyl-ammoniumchlorid;
- [2-(4-Amino-5-hydroxy-3-methyl-pyrazol-1-yl)-ethyl]-trimethylammoniumchlorid;
- 3-[2-(4-Amino-5-hydroxy-3-methyl-pyrazol-1-yl)-ethyl]-1-methyl-3H-imidazol-1-ium-chlorid;
- 3-[2-(4-Amhxo-5-hydroxy-pyrazol-1-yl)-ethyl]-1-methyl-3H-imidazol-1-ium-chlorid;
- 3-[2-(4,5-Diamino-1-methyl-1H-pyrazol-3-yl)-ethyl]-1-methyl-3H-imidazol-1-ium-chlorid;
- und ihre Additionssalze mit einer Säure oder einer Base und ihre gegebenenfalls existierenden tautomeren Formen.

7. Verbindungen nach Anspruch 6, **dadurch gekennzeichnet, dass** sie ausgewählt sind unter:
- [3-(4-,Amino-2,5-dimethyl-2H-pyrazol-3-ylamino)-propyl]-trimethyl-ammoniumchlorid;
- [3-(4-Amino-2,5-dimethyl-2H-pyrazol-3-ylamino)-propyl]-(2-hydroxy-ethyl)-dimethyl-ammoniumchlorid;
- 3-[3-(4-Amino-2,5-dimethyl-2H-pyrazol-3-ylamino)-propyl]-1-(2-hydroxy-ethyl) 3H-imidazol-1-ium-chlorid;
- 3-[(4-Amino-2H-pyrazol-3-ylcarbamoyl)-methyl]-1-methyl-3H-imidazol-1-ium-chlorid;
- 3-[2-(4,5-Diamino-3-methyl-pyrazol-1-yl)-ethyl]-1-methyl-3H-imidazol-1-ium-chlorid;
- 3-[2-(4,5-Diamino-1-methyl-1H-pyrazol-3-yl)-ethyl)-1-methyl-3H-imidazol-1-ium-chlorid;
- und ihre Additionssalze mit einer Säure oder einer Base und ihre gegebenenfalls existierenden tautomeren Formen.

8. Verbindungen nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten und Acetaten und die Additionssalze mit einer Base unter den Salzen ausgewählt sind, die mit Natriumhydroxid, Kaliumhydroxid, Ammoniak oder Aminen erhalten werden.

9. Verwendung von Verbindungen der Formel (I) nach einem der Ansprüche 1 bis 8 als Oxidationsbasen zum oxidativen Färben von Keratinfasern.

10. Zusammensetzung zum oxidativen Färben von Keratinfasern, **dadurch gekennzeichnet, dass** sie in einem zum Färben geeigneten Medium mindestens eine Verbindung der Formel (I) nach einem der Ansprüche 1 bis 8 als Oxidationsbase enthält.

11. Zusammensetzung nach Anspruch 10, **dadurch gekennzeichnet, dass** die Verbindung(en) der Formel (I) 0,0005 bis 12 Gew.-% des Gesamtgewichts der Farbmittelzusammensetzung ausmachen.

12. Zusammensetzung nach Anspruch 11, **dadurch gekennzeichnet, dass** die Verbindung(en) der Formel (I) 0,005 bis 6 Gew.-% des Gesamtgewichts der Farbmittelzusammensetzung ausmachen.

13. Zusammensetzung nach einem der Ansprüche 10 bis 12, **dadurch gekennzeichnet, dass** sie mindestens eine ergänzende Oxidationsbase enthält, die unter den p-Phenylendiaminen, Bisphenylalkylendiaminen, p-Aminophenolen, o-Aminophenolen und heterocyclischen Basen, die von den Verbindungen der Formel (I) verschieden sind, ausgewählt ist.

14. Zusammensetzung nach Anspruch 13, **dadurch gekennzeichnet, dass** die ergänzende(n) Oxidationsbase(n) 0,0005 bis 12 Gew.-% des Gesamtgewichts der Farbmittelzusammensetzung ausmachen.

15. Zusammensetzung nach einem der Ansprüche 10 bis 14, **dadurch gekennzeichnet, dass** sie mindestens einen Kuppler und/oder mindestens einen Direktfarbstoff enthält.

16. Zusammensetzung nach Anspruch 15, **dadurch gekennzeichnet, dass** der oder die Kuppler unter den m-Phenylendiaminen, m-Aminophenolen, m-Dihydroxybenzolen und heterocyclischen Kupplern und deren Additionssalzen mit einer Säure ausgewählt sind.

17. Zusammensetzung nach Anspruch 16, **dadurch gekennzeichnet, dass** der oder die Kuppler unter 2-Methyl-5-aminophenol, 5-N-(β-Hydroxyethyl)amino-2-methylphenol, 3-Aminophenol, 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis-(2,4-diaminophenoxy)-propan, Sesamol, α-Naphthol, 6-Hydroxyindol, 4-Hydroxyindol, 4-Hydroxy-N-methylindol, 6-Hydroxyindolin, 2,6-Dihydroxy-4-methylpyridin, 1-H-3-Methyl-pyrazol-5-on, 1-Phenyl-3-methyl-pyrazol-5-on und deren Additionssalzen mit einer Säure ausgewählt sind.

18. Zusammensetzung nach einem der Ansprüche 15 bis 17, **dadurch gekennzeichnet, dass** der oder die Kuppler etwa 0,0001 bis 10 Gew.-% des Gesamtgewichts der Farbmittelzusammensetzung ausmachen.

19. Zusammensetzung nach einem der Ansprüche 10 bis 18, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

20. Verfahren zum Färben von Keratinfasern und insbesondere menschlichen Keratinfasern, beispielsweise zum Färben der Haare, **dadurch gekennzeichnet, dass** auf die Fasern mindestens eine Farbmittelzusammensetzung nach einem der Ansprüche 10 bis 19 aufgebracht wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgebracht wird.

21. Verfahren nach Anspruch 20, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, und Enzymen ausgewählt ist.

22. Vorrichtung mit mehreren Abteilungen oder "Kit" zum Färben mit mehreren Abteilungen, wobei eine erste Abteilung eine Farbmittelzusammensetzung nach einem der Ansprüche 10 bis 19 und eine zweite Abteilung eine oxidierende Zusammensetzung enthält.

## Claims

1. Compounds of formula (I) below, the addition salts thereof with an acid or with a base, and the possible tautomeric forms thereof: 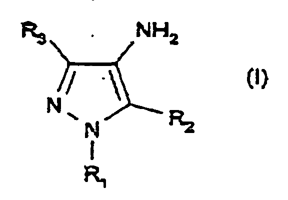 in which:
• R₁ represents a hydrogen atom; a group Z as defined below; a C₁-C₆ alkyl radical; a C₁-C₆ trifluoroalkyl radical; a C₁-C₆ monohydroxyalkyl radical; a C₂-C₆ polyhydroxyalkyl radical; an aryl (C₁-C₆)alkyl radical in which the aryl radical is a phenyl radical or a 5-or 6-membered aromatic heterocycle; a (C₁-C₆)alkoxy(C₁-C₆)alkyl radical; a (C₁-C₆)alkylthio-(C₁-C₆)alkyl radical; an amino (C₁-C₆)alkylcarbonyl-(C₁-C₆)alkyl radical; an N-(C₁-C₆)alkylamino(C₁-C₆)-alkylcarbonyl(C₁-C₆)alkyl radical; an N,N-di(C₁-C₆)-alkylamino(C₁-C₆)alkylcarbonyl(C₁-C₆)alkyl radical; a C₁-C₆ aminosulphonylalkyl radical; an N-(C₁-C₆)alkylaminosulphonyl(C₁-C₆)alkyl radical; an N,N-di(C₁-C₆)-alkylaminosulphonyl(C₁-C₆)alkyl radical;
• R₂ and R₃, which may be identical or different, represent a group -NHR₄; a hydroxyl radical ; a halogen atom; a nitro radical; a cyano radical; a carboxyl radical; a C₁-C₆ alkylcarboxyl radical; a carbamyl radical; an N-(C₁-C₆)alkylcarbamyl radical; an N,N-di(C₁-C₆)alkylcarbamyl radical; a C₁-C₆ alkoxy radical; a C₁-C₆ thioalkyl radical; or one of the meanings given above for R₁; it being understood that at least one of the radicals R₂ and R₃ represents a group -NHR₄ or a hydroxyl radical;
• R₄ represents a hydrogen atom; a group Z as defined below; a C₁-C₆ alkyl radical; a C₁-C₆ monohydroxyalkyl radical; a C₂-C₆ polyhydroxyalkyl radical; a (C₁-C₆)-alkoxy(C₁-C₆)alkyl radical; a benzyl radical; a cyano(C₁-C₆)alkyl radical; a carbamyl(C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a thiocarbamyl(C₁-C₆)alkyl radical; a C₁-C₆ trifluoroalkyl radical; a C₁-C₆ sulphoalkyl radical; a (C₁-C₆)alkylcarboxy(C₁-C₆) alkyl radical; a (C₁-C₆)-alkylsulphinyl(C₁-C₆)alkyl radical; a C₁-C₆ aminosulphonylalkyl radical; an N-(C₁-C₆)alkylaminosulphonyl(C₁-C₆)alkyl radical; an N,N-di(C₁-C₆)alkylaminosulphonyl(C₁-C₆)alkyl radical; a C₁-C₆ aminoalkyl radical; a C₁-C₆ aminoalkyl radical in which the amine is substituted with one or two radicals, which may be identical or different, chosen from C₁-C₆ alkyl, C₁-C₆ monohydroxyalkyl, C₂-C₆ polyhydroxyalkyl, (C₁-C₆)alkylcarbonyl, C₁-C₆ alkylsulphonyl, formyl and trifluoro-(C₁-C₆)alkylcarbonyl radicals; the amine of the C₁-C₆ aminoalkyl radical may also be substituted with two radicals forming, together with the nitrogen atom of the said amine, a saturated or unsaturated 5- or 6-membered ring which may contain one or more hetero atoms chosen from nitrogen, oxygen and sulphur;
• Z is chosen from the unsaturated cationic groups of formulae (II) and (III) below, and the saturated cationic groups of formula (IV) below: 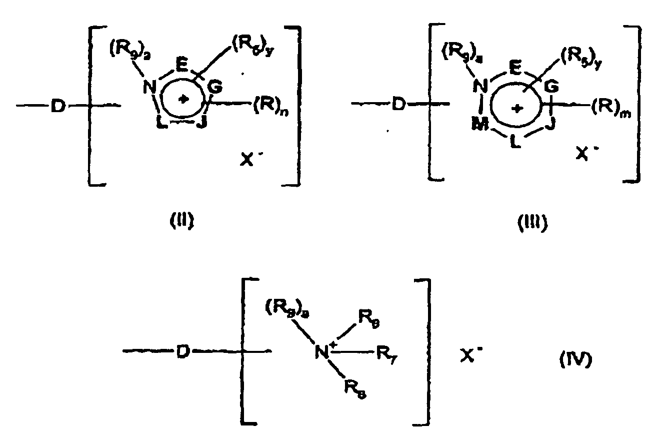 in which:
• D is a linker arm which represents a linear or branched alkyl chain preferably containing from 1 to 14 carbon atoms, which can be substituted with one or more hydroxyl or C₁-C₆ alkoxy radicals, and which can bear one or more ketone functions;
• the ring members E, G, J, L and M, which may be identical or different, represent a carbon or nitrogen atom;
• n is an integer between 0 and 4 inclusive;
• m is an integer between 0 and 5 inclusive;
• the radicals R, which may be identical or different, represent a halogen atom; a hydroxyl radical; a C₁-C₆ alkyl radical; a C₁-C₆ monohydroxyalkyl radical; a C₂-C₆ polyhydroxyalkyl radical; a nitro radical; a cyano radical: a cyano(C₁-C₆)alkyl radical; a C₁-C₆ alkoxy radical; a tri(C₁-C₆)alkylsilane(C₁-C₆)alkyl radical; an amido radical; an aldehydo radical; a carboxyl radical; a (C₁-C₆)alkylcarbonyl radical; a thio radical; a C₁-C₆ thioalkyl radical; a C₁-C₆ alkylthio radical; an amino radical; an amino radical protected with a (C₁-C₆)alkylcarbonyl or C₁-C₆ alkylsulphonyl radical; a group NHR" or NR"R'" in which R" and R"', which may be identical or different, represent a C₁-C₆ alkyl radical, a C₁-C₆ monohydroxyalkyl radical or a C₂-C₆ polyhydroxyalkyl radical;
• R₅ represents a C₁-C₆ alkyl radical; a C₁-C₆ monohydroxyalkyl radical; a C₂-C₆ polyhydroxyalkyl radical; a cyano(C₁-C₆)alkyl radical; a tri(C₁-C₆)alkylsilane(C₁-C₆)alkyl radical; a (C₁-C₆)alkoxy(C₁-C₆)alkyl radical; a carbamyl(C₁-C₆)-alkyl radical; a (C₁-C₆)alkylcarboxy(C₁-C₆)alkyl radical; a benzyl radical;
• R₆, R₇ and R₈, which may be identical or different, represent a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a cyano(C₁-C₆)alkyl radical; a benzyl radical; a C₁-C₆ amidoalkyl radical; a tri(C₁-C₆) alkylsilane(C₁-C₆)alkyl radical or a C₁-C₆ aminoalkyl radical in which the amine is protected with a (C₁-C₆)alkylcarbonyl or C₁-C₆ alkylsulphonyl radical; two of the radicals R₆, R₇ and R₈ can together also form, with the nitrogen atom to which they are attached, a saturated 5- or 6-membered carbon ring or a ring containing one or more hetero atoms, it being possible for the said ring to be unsubstituted or substituted with a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri(C₁-C₆) alkylsilane(C₁-C₆)alkyl radical, an amido radical, an aldehydo radical, a carboxyl radical, a keto(C₁-C₆)alkyl radical, a thio radical, a C₁-C₆ thioalkyl radical, a C₁-C₆ alkylthio radical, an amino radical or an amino radical protected with a (C₁-C₆)alkylcarbonyl or C₁-C₆ alkylsulphonyl radical;
• R₉ represents a C₁-C₆ alkyl radical; a C₁-C₆ monohydroxyalkyl radical; a C₂-C₆ polyhydroxyalkyl radical; a benzyl radical; a C₁-C₆ aminoalkyl radical; a C₁-C₆ aminoalkyl radical in which the amine is protected with a (C₁-C₆)alkylcarbonyl or C₁-C₆ alkylsulphonyl radical; a carboxy(C₁-C₆)alkyl radical; a cyano (C₁-C₆)alkyl radical; a carbamyl (C₁-C₆)alkyl radical; a C₁-C₆ trifluoroalkyl radical; a tri(C₁-C₆)alkylsilane(C₁-C₆)alkyl radical; a C₁-C₆ sulphonamidoalkyl radical; a (C₁-C₆)alkylcarboxy(C₁-C₆)alkyl radical; a (C₁-C₆) alkylsulphinyl(C₁-C₆)alkyl radical; a (C₁-C₆) alkylsulphonyl(C₁-C₆)alkyl radical; a (C₁-C₆)alkylketo(C₁-C₆)alkyl radical; an N-(C₁-C₆)alkylcarbamyl(C₁-C₆)alkyl radical; an N-(C₁-C₆)alkylsulphonamido(C₁-C₆)alkyl radical;
• a and y are integers equal to 0 or 1; with the following conditions:
- in the unsaturated cationic groups of formula (II):
- when a = 0, the linker arm D is attached to the nitrogen atom,
- when a = 1, the linker arm D is attached to one of the ring members E, G, J or L,
- y can take the value 1 only:
1) when the ring members E, G, J and L simultaneously represent a carbon atom and when the radical R₅ is borne by the nitrogen atom of the unsaturated ring; or alternatively
2) when at least one of the ring members E, G, J and L represents a nitrogen atom to which the radical R₅ is attached;
- in the unsaturated cationic groups of formula (III) :
- when a = 0, the linker arm D is attached to the nitrogen atom,
- when a = 1, the linker arm D is attached to one of the ring members E, G, J, L or M,
- y can take the value 1 only when at least one of the ring members E, G, J, L and M represents a divalent atom and when the radical R₅ is borne by the nitrogen atom of the unsaturated ring;
- in the cationic groups of formula (IV):
- when a = 0, then the linker arm D is attached to the nitrogen atom bearing the radicals R₆ to R₈,
- when a = 1, then two of the radicals R₆ to R₈ form, together with the nitrogen atom to which they are attached, a saturated 5- or 6-membered ring as defined above, and the linker arm D is borne by a carbon atom of the said saturated ring;
• X⁻ represents a monovalent or divalent anion;
it being understood that the number of cationic groups Z is equal to 1.

2. Compounds according to claim 1, **characterized in that** the rings of the unsaturated groups Z of formula (II) are chosen from pyrrole, imidazole, pyrazole and triazole rings.

3. Compounds according to claim 1, **characterized in that** the rings of the unsaturated groups Z of formula (III) are chosen from pyridine, pyrimidine, pyrazine and triazine rings.

4. Compounds according to any one of the preceding claims, **characterized in that** two of the radicals R₆, R₇ and R₈ form a pyrrolidine ring, a piperidine ring, a piperazine ring or a morpholine ring.

5. Compounds according to any one of the preceding claims, **characterized in that** X⁻ is chosen from a halogen atom, a hydroxide, a hydrogenosulphate and a C₁-C₆ alkyl sulphate.

6. Compounds according to any one of the preceding claims, **characterized in that** they are chosen from:
- [3-(4-amino-2,5-dimethyl-2H-pyrazol-3-ylamino)propyl]-trimethylammonium chloride;
- [3-(4-amino-2,5-dimethyl-2H-pyrazol-3-ylamino)propyl]-(2-hydroxyethyl)dimethylammonium chloride;
- 3-[3-(4-amino-2,5-dimethyl-2H-pyrazol-3-ylamino)-propyl]-1-(2-hydroxyethyl)-3H-imidazol-1-ium chloride;
- 3-[(4-amino-2H-pyrazol-3-ylcarbamoyl)methyl]-1-methyl-3H-imidazol-1-ium chloride;
- 3-[2-(4,5-diamino-3-methylpyrazol-1-yl)ethyl]-1-methyl-3H-imidazol-1-ium chloride;
- 3-[2,(4,5-diaminopyrazol-1-yl)ethyl)-1-methyl-3H-imidazol-1-ium chloride;
- [2-(4,5-diamino-3-methylpyrazol-1-yl)ethyl]trimethylammonium chloride;
- [2-(4,5-diaminopyrazol-1-yl)ethyl]trimethylammonium chloride;
- [2-(4-amino-5-hydroxypyrazol-1-yl)ethyl]trimethylammonium chloride;
- [2-(4-amino-5-hydroxy-3-methylpyrazol-1-yl)ethyl]-trimethylammonium chloride;
- 3-[2-(4-amino-5-hydroxy-3-methylpyrazol-1-yl)ethyl]-1-methyl-3H-imidazol-1-ium chloride;
- 3-[2-(4-amino-5-hydroxypyrazol-1-yl)ethyl]-1-methyl-3H-imidazol-1-ium chloride;
- 3-[2-(4,5-diamino-1-methyl-1H-pyrazol-3-yl)ethyl]-1-methyl-3H-imidazol-1-ium chloride,
and the addition salts thereof with an acid or with a base, and the possible tautomeric forms thereof.

7. Compounds according to Claim 6, **characterized in that** they are chosen from:
- [3-(4-amino-2,5-dimethyl-2H-pyrazol-3-ylamino)propyl]-trimethylammonium chloride;
- [3-(4-amino-2,5-dimethyl-2H-pyrazol-3-ylamino)propyl]-(2-hydroxyethyl)dimethylammonium chloride;
- 3-[3-(4-amino-2,5-dimethyl-2H-pyrazol-3-ylamino)-propyl]-1-(2-hydroxyethyl)-3H-imidazol-1-ium chloride;
- 3-[(4-amino-2H-pyrazol-3-ylcarbamoyl)methyl]-1-methyl-3H-imidazol-1-ium chloride;
- 3-[2-(4,5-diamino-3-methylpyrazol-1-yl)ethyl]-1-methyl-3H-imidazol-1-ium chloride;
- 3-[2-(4,5-diamino-1-methyl-1H-pyrazol-3-yl)ethyl]-1-methyl-3H-imidazol-1-ium chloride;
and the addition salts thereof with an acid or with a base, and the possible tautomeric forms thereof.

8. Compounds according to any one of the preceding claims, **characterized in that** the addition salts with an acid are chosen from the hydrochlorides, hydrobromides, sulphates, citrates, succinates, tartrates, lactates and acetates and **in that** the addition salts with a base are chosen from those obtained with sodium hydroxide, potassium hydroxide, aqueous ammonia and amines.

9. Use of the compounds of formula (I) as defined in any one of Claims 1 to 8, as oxidation bases for the oxidation dyeing of keratin fibres.

10. Composition for the oxidation dyeing of keratin fibres, **characterized in that** it comprises as oxidation base, in a medium which is suitable for dyeing, at least one compound of formula (I) as defined in any one of Claims 1 to 8.

11. Composition according to Claim 10, **characterized in that** the compound(s) of formula (I) repxesent(s) from 0.0005 to 12% by weight relative to the total weight of the dye composition.

12. Composition according to Claim 11, **characterized in that** the compound(s) of formula (I) represent(s) from 0.005 to 6% by weight relative to the total weight of the dye composition.

13. Composition according to any one of Claims 10 to 12, **characterized in that** it contains at least one additional oxidation base chosen from para-phenylenediamines, bis(phenyl)alkylenediamines, para-aminophenols, ortho-aminophenols and heterocyclic bases other than the compounds of formula (I).

14. Composition according to Claim 13, **characterized in that** the additional oxidation base(s) represent(s) from 0.0005% to 12% by weight relative to the total weight of the dye composition.

15. Composition according to any one of Claims 10 to 14, **characterized in that** it contains at least one coupler and/or at least one direct dye.

16. Composition according to Claim 15, **characterized in that** the coupler(s) is (are) chosen from meta-phenylenediamines, meta-aminophenols, meta-diphenols and heterocyclic couplers, and the addition salts thereof with an acid.

17. Composition according to Claim 16, **characterized in that** the coupler(s) is (are) chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-rnethylindole, 6-hydroxyindoline, 2,6-dihydroxy-4-methylpyridine, 1H-3-methylpyrazol-5-one and 1-phenyl-3-methylpyrazol-5-one, and the addition salts thereof with an acid.

18. Composition according to any one of claims 15 to 17, **characterized in that** the coupler(s) represent(s) from 0.0001 to 10% by weight relative to the total weight of the dye composition.

19. Composition according to any one of Claims 10 to 18, **characterized in that** the addition salts with an acid are chosen from the hydrochlorides, hydrobromides, sulphates, citrates, succinates, tartrates, lactates and acetates.

20. Process for dyeing keratin fibres, and in particular human keratin fibres such as the hair, **characterized in that** at least one dye composition as defined in any one of Claims 10 to 19 is applied to these fibres and the colour is developed at acidic, neutral or alkaline pH using an oxidizing agent which is added to the dye composition just at the time of use, or which is present in an oxidizing composition which is applied simultaneously or sequentially in a separate manner.

21. Process according to Claim 20, **characterized in that** the oxidizing agent is chosen from hydrogen peroxide, urea peroxide, alkali metal bromates and persalts such as perborates and persulphates, and enzymes.

22. Multi-compartment dyeing device or multi-compartment dyeing kit, a first compartment of which contains a dye composition as defined in any one of Claims 10 to 19, and a second compartment of which contains an oxidizing composition.
